Europäisches Patentamt

European Patent Office

Office européen des brevets

(19)

(11) Veröffentlichungsnummer. **0 158 092**

**A1**

(12) # EUROPÄISCHE PATENTANMELDUNG

(21) Anmeldenummer: 85102235.0

(22) Anmeldetag: 28.02.85

(51) Int. Cl.⁴: **A 61 L 15/00**
**A 01 N 25/10, A 01 N 59/20**

(30) Priorität: 06.03.84 DE 3408130

(43) Veröffentlichungstag der Anmeldung:
16.10.85 Patentblatt 85/42

(84) Benannte Vertragsstaaten:
AT BE CH DE FR GB IT LI LU NL SE

(71) Anmelder: Henkel Kommanditgesellschaft auf Aktien
Postfach 1100 Henkelstrasse 67
D-4000 Düsseldorf-Holthausen(DE)

(72) Erfinder: Sustmann, Scarlet, Dr.
Am Nachtigallenwäldchen 34
D-4060 Viersen 12(DE)

(72) Erfinder: Marini, Ingo Gerhard, Dr.
Hauptstrasse 40
A-4860 Lenzing(AT)

(54) Sanitäre Hygienemittel.

(57) Sanitäre Hygienemittel, z.B. Tampons, Binden und Slipeinlagen, bestehend aus einem Fasermaterial aus Cellulosefasern, die durch salzbildende, anionische Gruppen, bevorzugt durch Carboxymethylgruppen, modifiziert sind und die über diese Gruppen an die Faser gebundenes Kupfer enthalten. Der Substitutionsgrad der Cellulosefasern sollte wenigstens so hoch sein, daß 0,1 bis 3,0 Gew.-% Kupfer gebunden werden kann.

EP 0 158 092 A1

Croydon Printing Company Ltd.

Henkelstraße 67
4ooo Düsseldorf, den 27.2.1984

HENKEL KGaA
ZR-FE/Patente

Dr.JG/Po

— 1 —

P a t e n t a n m e l d u n g

D 6938 EP

"Sanitäre Hygienemittel"

Die Erfindung betrifft sanitäre Hygienemittel, z.B. Menstrualtampons und Binden für die Frauenhygiene und Slipeinlagen aus einem neuen, desodorierenden und mikrobistatischen Fasermaterial.

Es ist bekannt, daß in sanitären Hygienemitteln, die aus absorbierendem Fasermaterial bestehen und zur Aufnahmen von Körpersekreten, z.B. von Blut, Urin, Menstrualflüssigkeit dienen, bereits nach kurzer Zeit bakterielle Zersetzung des Sekrets durch ubiquitär vorhandene Bakterien und in Verbindung damit eine unangenehme Geruchsentwicklung erfolgt. Durch das Wachstum pathogener Keime werden darüber hinaus ernsthafte gesundheitliche Gefahren bedingt. Es hat nicht an Versuchen gefehlt, sanitäre Hygienemittel aus absorbierenden Fasermaterialien desodorierend und mikrobistatisch auszurüsten. Es wurden für diesen Zweck verschiedene Mikrobizide und Desodorantien vorgeschlagen, die zur Unterdrückung des Schweißgeruchs geeignet sind.

Die Unterdrückung des Zersetzungsgeruchs von Blut, Urin oder Menstrualflüssigkeit stellt jedoch ein vergleichsweise schwieriges Problem dar, insbesondere da die üblichen antimikrobiellen Wirkstoffe nicht fest auf der

...

- 2 -

Faser verankert sind und durch die genannten Sekrete leicht mobilisiert werden. Man hat auch schon vorgeschlagen, Kupferverbindungen zur Ausrüstung von Fasermaterialien für sanitäre Hygienemittel einzusetzen.

Aus DE-OS 31 35 410 ist z.B. ein absorbierender Körper aus Cellulose-Faservlies oder Watte bekannt, der mit der Lösung eines Kupfersalzes besprüht oder aus einer mit einer solchen Lösung behandelten Faser oder Watte angefertigt ist. Dieses Material hat den Nachteil, daß das Kupfersalz nicht gleichmäßig in dem absorbierenden Körper verteilt ist, daß das Kupfersalz nach dem Trocknen teilweise auskristallisiert, was bei der Verarbeitung des Fasermaterials zu Schwierigkeiten führt und daß das Kupfersalz sich in Gegenwart von Wasser, z.B. in einer nassen Windel oder in einem feuchten Tampon löst und so in relativ hoher Konzentration an die Hautoberfläche gelangen und dort zu unerwünschten Reaktionen führen kann.

Aus EP-A 19 371 ist ein blutkoagulierendes Material bekannt, welches aus einem wasserquellbaren, kovalent vernetzten, anionischen Polyelektrolyten, z.B. aus vernetzter Carboxymethylcellulose besteht, der mit Übergangsmetallionen, z.B. mit Kupfer-ionen ausgerüstet ist. Solche Materialien sind aber selbst nicht faserförmig bzw. nicht zu Fasern, Watte oder Vliesstoffen zu verarbeiten, so daß sie bei ihrer Anwendung in Hygienemitteln mit einem Fasermaterial als Träger verarbeitet werden müssen. Sowohl die Herstellung als auch die Verarbeitung dieses Materials ist sehr aufwendig.

...

- 3 -

Es bestand daher die Aufgabe, sanitäre Hygienemittel auf Basis eines saugfähigen Fasermaterials zu entwickeln, welches dauerhaft, also nicht auswaschbar, desodorierend und mikrobistatisch ausgerüstet ist und auf einfache Weise herstellbar und verarbeitbar ist.

Es wurde gefunden, daß diese Aufgabe auf überraschend elegante Weise erfüllt wird durch sanitäre Hygienemittel, die ganz oder teilweise aus einem absorbierenden Fasermaterial bestehen, welches aus durch anionische, salzbildende Gruppen modifizierten Cellulosefasern besteht und über die anionischen Gruppen an die Faser gebundenes Kupfer enthält. Unter Cellulosefasern werden vor allem Zellstoff, Baumwolle und Viskosefasern verstanden.

Anionische, salzbildende Gruppen lassen sich auf vielerlei Weise in das Cellulosemolekül einbauen. Solche anionisch modifizierte Cellulosefasern sind bekannt, einige davon auch im Handel erhältlich. Geeignet sind z.B. Cellulosefasern, die über den Sauerstoff an die Anhydroglukoseeinheiten gebundene Gruppen der allgemeinen Formel $- PO_3H^{(-)}$, $-(CH_2)_n - PO_3H^{(-)}$, $-(CH_2)_n - SO_3^{(-)}$ $(CH_2)_n - COO^{(-)}$ tragen, wobei n einen Wert von 1 - 3 haben kann. Bekannte Cellulosederivate dieser Art sind z.B. Cellulosephosphat, welches durch Veresterung von Cellulose mit Phosphorsäure zugänglich ist, Phosphonoethylcellulose, die durch Veretherung von Alkalicellulose mit Chlorethylphosphonat erhältlich ist, Phosphonomethylcellulose, die durch Veretherung von Cellulose mit Chlormethylphosphonat hergestellt werden kann, Sulfoethylcellulose, die durch Veretherung von Cellulose mit Chlorethansulfonat zugänglich ist und die analog herstellbare Sulfomethylcellulose und Sulfopropylcellulose. Durch Veretherung mit 1-Chlor-2-hydroxypropansulfonat

...

- 4 -

ist die 1-Sulfo-2-hydroxypropylcellulose erhältlich.

Die Einführung von Carboxylgruppen in das Cellulosemolekül ist auf zwei prinzipiell verschiedenen Wegen
möglich:

- durch physikalische Inkorporation von Carboxylgruppen
  tragenden Verbindungen in die Viskose, d.h. in eine
  als Cellulosexanthogenat gelöste Cellulose, unter
  Bildung von sogenannter inkorporierter Viskosefasern (Alloy Fibers) oder

- durch chemische Umsetzung (Veretherung) der faserbildenden Cellulose mit Carboxylgruppen tragenden
  Reagentien unter Bildung von einheitlich mit z.B.
  Carboxyalkylgruppen der Formel $- (CH_2)_n - COOH$, in
  der n einen Wert von 1 - 3 haben kann, modifizierten Cellulosefasern.

Die physikalische Inkorporation Carboxylgruppen tragender Verbindungen in die Viskose wird z.B. durch
Beimischen von Alkalisalzen von Acrylsäure-Homopolymerisaten, Acrylsäure-Methacrylsäure-Copolymerisaten,
Methylvinylether-Maleinsäureanhydrid-Copolymerisaten,
Alginsäure oder Carboxymethylcellulose zur Viskoselösung und anschließendes Verspinnen in üblicher Weise in ein Fällungsbad erreicht. Handelsübliche Fasern dieses Typs sind z.B. die ABSORBIT[R] - Fasern
von Fa. Enka, die eine Mischfaser aus Viskose und Acrylsäure-Methacrylsäure-Copolymerisat darstellen. Solche

...

Fasern sind nicht einheitlich modifiziert, sondern setzen sich aus modifizierten und unsubstituierten Faserfragmenten zusammen.

Einheitlich chemisch mit Carboxyalkylgruppen modifizierte Cellulosefasern sind für die Herstellung der erfindungsgemäßen Fasermaterialien besonders bevorzugt. In solchen Fasern ist die gesamte faserbildende Cellulose gleichmäßig modifiziert. Sie werden z.B. dadurch erhalten, daß man Cellulosefasern direkt nach Überführung in die Alkalicellulose mit z.B. Natriumchloracetat carboxymethyliert. Die so modifizierte Cellulose kann über das Viskose-Spinnverfahren in ihrer Faserstruktur verbessert werden. Man kann aber auch eine über das Viskose-Spinnverfahren regenerierte Viskosefaser anschliessend mit Chloressigsäure carboxymethylieren. Eine dritte Möglichkeit, zu einheitlich mit Carboxyalkylgruppen modifizierten Cellulosefasern zu gelangen, besteht darin, daß man während der Xanthogenierung der Viskoselösung z.B. Na-Chloracetat zusetzt und dann die carboxymethylierte Viskose wie üblich verspinnt. Solche einheitlich mit Carboxymethylgruppe modifizierte Viskosefasern sind im Handel erhältlich, z.B. unter der Bezeichnung VISCOSORB 1 N von Fa. Chemiefaser Lenzing.

Setzt man der Viskose-Lösung während der Xanthogenierung z.B. Acrylnitril zu, so erhält man nach Ablauf des Viskosespinnprozesses Viskosefasern, die aus niedrig substituierter Carboxyethylcellulose bestehen. Solche Fasern sind unter der Bezeichnung BAR-Fieber (Bondable Avisco Rayon) der FMC-Corporation im Handel.

...

0158092

- 6 -

Andere zur einheitlichen Modifizierung der Viskose durch Zusatz zur Viskoselösung während der Xanthogenierung geeignete Reagenzien sind z.B. Natrium-Vinylsulfonat, Na-Chlor-methansulfonat, Na-Chlormethanphosphonat. Man erhält auf diese Weise einheitlich chemisch modifizierte Viskosefasern mit Sulfoethylgruppen, Sulfomethylgruppen und Phosphonomethylgruppen.

Zur Herstellung der erfindungsgemäßen sanitären Hygienemittel ist jedoch eine mit Carboxylgruppen, insbesondere mit Carboxymethylgruppen einheitlich chemisch modifizierte Cellulosefaser besonders bevorzugt, insbesondere ein Fasermaterial, welches aus einer durch das Viskose-Spinnverfahren regenerierten, mit Carboxymethylgruppen modifizierten Cellulose erhalten und mit einer Kupfersalzlösung behandelt wird.

Der Substitutionsgrad der für das erfindungsgemäß zu verwendende Fasermaterial geeigneten Cellulosederivate sollte in bezug auf die anionischen, salzbildenden Gruppen so hoch sein, daß sie 0,1 bis 3,0 Gew.-% Kupfer, bezogen auf das Gewicht des Fasermaterials, binden können. Die für das erfindungsgemäße Fasermaterial am besten geeigneten, mit Carboxymethylgruppen modifizierten Viskosefasern weisen einen Substitutionsgrad von 0,01 bis 0,3 auf, d.h. sie enthalten im Mittel etwa 0,01 bis 0,3 Carboxymethylgruppen pro Anhydroglukoseeinheit. Der Gehalt an gebundenem Kupfer sollte von 0,2 - 2,0 Gew.-%, bevorzugt von 0,6 - 1,6 Gew.-% des Fasermaterials ausmachen.

...

- 7 -

Es hat sich weiterhin als vorteilhaft erwiesen, wenn das erfindungsgemäß zu verwendende Fasermaterial einen Faser-pH-Wert, gemessen nach DIN 54275, von 4 - 5 aufweist. Ein solcher Faser-pH-Wert bewirkt, daß die erfindungsgemäßen Fasermaterialien einen gewissen Puffereffekt gegenüber aufgesaugten Körperflüssigkeiten haben und so den physiologisch günstigen, schwach sauren pH-Wert der Hautoberfläche herstellen, wodurch Reizungen und Anfälligkeit gegenüber Erkrankungen vermieden werden.

Das für die erfindungsgemäßen sanitären Hygienemittel zu verwendende Fasermaterial sollte in einer weiteren bevorzugten Ausführungsform ein hohes Wasserrückhaltevermögen, gemessen nach DIN 53814, von mindestens 80 % aufweisen.

Die Herstellung des erfindungsgemäß zu verwendenden Fasermaterials ist aus bekannten Fasern mit anionischen, salzbildende Gruppen enthaltenden Fasern in einfacher Weise dadurch möglich, daß man

- die anionische, salzbildende Gruppen enthaltenden Fasern mit einer wäßrigen Kupfer-II-salzlösung behandelt und

- die Fasern mit Wasser weitgehend salzfrei wäscht und trocknet.

Das bevorzugt zu verwendende Fasermaterial mit hohem Wasserrückhaltevermögen läßt sich sehr leicht dadurch herstellen, daß man eine Carboxymethyl-Viskosefaser mit einem Substitutionsgrad von 0,01 - 0,3 und mit ent-

...

sprechend hohem Wasserrückhaltevermögen, z.B. die handelsüblichen Fasern Viscosorb[R] 1S mit einem Wasserrückhaltevermögen von ca. 200 % und einem Substitutionsgrad von ca.0,1 entweder in der Natriumsalzform oder nach Überführung in die freie Säureform in das beschriebene Verfahren einsetzt. Als wäßrige Kupfer-II-salzlösung eignet sich z.B. eine Lösung von 1 - 20 g/l $CuSO_4$ · $5 H_2O$ in Wasser. Die Behandlung erfolgt im allgemeinen ohne Wärmezufuhr über einen Zeitraum von wenigstens 1 Minute und ist in höchstens 1 Stunde beendet. Danach wird die Kupfersalzlösung, z.B. durch Abpressen, von der Faser abgetrennt, die Faser mit Wasser so lange gewaschen, bis das Waschwasser weitgehend frei von Sulfationen ist, das Wasser, z.B. durch Abpressen, von der Faser abgetrennt und die Faser im Luftstrom getrocknet. Die Erzeugung eines erfindungsgemäß zu verwendenden Fasermaterials mit einem Faser-pH-Wert von 4 - 5 läßt sich nach diesem Verfahren leicht dadurch erreichen, daß man eine auf einen pH-Wert von 4 - 5 eingestellte Kupfer-II-salzlösung einsetzt.

Das erfindungsgemäß zu verwendende Fasermaterial weist eine von der Menge des gebundenen Kupfers abhängige, mehr oder weniger ausgeprägte Blaufärbung auf, die unter Anwendungsbedingungen nicht auswaschbar ist. Die Farbe ist für die erfindungsgemäße Verwendung in sanitären Hygienemitteln nicht störend und entspricht durchaus hygienischen Vorstellungen.

Die beschriebenen Fasern lassen sich nach üblicher Verfahrensweise zu Hygienemitteln, insbesondere Men-

...

HENKEL KGaA
ZR-FE/Patente

D158092

- 9 -

strualtampons, Hygienebinden, Slipeinlagen, aber auch zu Babywindeln, Tupfern und Tampons für die Dental- und Operationstechnik und generell zu saugfähigen hygienischen Einwegartikeln verarbeiten. Die mit Kupfer ausgerüsteten Fasermaterialien können entweder allein oder mit anderen Fasern gemeinsam zu erfindungsgemässen Hygienemitteln verarbeitet werden. Bevorzugt werden für die erfindungsgemäßen Hygienemittel ausschließlich Fasermaterialien der beschriebenen Art verwendet. Solche Hygienemittel haben den großen Vorteil, daß Bakterien, insbesondere solche, die im Intimbereich auftreten, z.B. Escherichia coli, Staphylococcus aureus und Candida albicans in solchen Hygienemitteln selbst unter optimalen Brutbedingungen keine Vermehrung zeigen. Auch weisen mit den erfindungsgemäß zu verwendenden Fasermaterialien beschickte Nährkulturen selbst nach dreitägiger Inkubation nicht den unangenehmen, charakteristischen Geruch auf, den entsprechende Kulturen, die mit Normalwatte beschichtet sind, aufweisen.

Da das Kupfer an die anionischen und elektronegativen Gruppen der Fasern fest gebunden ist, besteht nicht die Gefahr, daß Kupfersalz durch die Körpersekrete gelöst in höherer Konzentration auf die Haut oder gar auf Schleimhäute gelangen und dort zu toxischen Nebenwirkungen führt.

Die folgenden Beispiele sollen den Gegenstand der Erfindung näher erläutern:

B e i s p i e l e

<u>Herstellung des desodorierenden und mikrobistatischen
Fasermaterials</u>

Beispiel 1

1 kg carboxymethylierter Viskosefasern des Typs Viscosorb$^{(R)}$ 1S mit einem Substitutionsgrad von 0,10
wurde mit 20 l einer Lösung von 20 g $CuSO_4$ $\cdot$ $5H_2O$ in
1000 ml Wasser, deren pH-Wert mit verdünnter Schwefelsäure auf 5 eingestellt war, 30 Minuten bei Raumtemperatur (20$^o$C) behandelt. Anschließend wurde das Fasermaterial auf eine Feuchte von 200 % abgepreßt und solange mit Wasser gewaschen, bis das Waschwasser sulfatfrei war. Danach wurde wieder auf eine Feuchte von 200 %
abgepreßt und 4 Stunden im Umlufttrockenschrank bei
105$^o$C getrocknet. Das erhaltene Fasermaterial hatte
einen Faser-pH-Wert von 5,6 (nach DIN 54275), einen
Kupfer-Gehalt von 1,45 Gew.-% und einem Wasserrückhaltevermögen von 87 % (nach DIN 53814).

Beispiel 2

1 kg carboxymethylierter Viskosefasern der Type Viscosorb$^{(R)}$ 1S mit einem Substitutionsgrad von 0,10
wurde durch Behandlung mit einer 0,2 %igen wäßrigen
Salzsäurelösung (30 Minuten bei Raumtemperatur von 20$^o$C)
und Auswaschen der überschüssigen Salzsäure in die
Säureform überführt. Die auf eine Feuchte von ca. 200 %
abgepreßten Fasern wurden dann mit einer Lösung von
5 g $CuSO_4$ $\cdot$ $5H_2O$ in 1000 ml Wasser, deren pH-Wert auf
4,5 mit verdünnter Schwefelsäure eingestellt war, 30 Minuten bei Raumtemperatur von 20$^o$C behandelt. Die Auf-

. . .

arbeitung erfolgte analog Beispiel 1. Das erhaltene Fasermaterial hatte einen Faser-pH-Wert von 4,2 (nach DIN 54275), einen Kupfer-Gehalt von 0,64 Gew.-% und einem Wasserrückhaltevermögen von 109,5 (nach DIN 53814).

Beispiel 3

Herstellung von Tampons aus dem desodorierenden und mikrobistatischen Fasermaterial gemäß Beispiel 1.

Das Fasermaterial wurde, wie zur Tamponherstellung üblich, an einer Krempel aufgelöst und zu einem Watteband mit einer auf die Fläche bezogenen Masse von ca. 630 g/m$^2$ verarbeitet. Die Verfestigung erfolgte durch Vernadelung.

Auf Wattebandabschnitte (40 x 90 mm) wurde die Rückholkordel in Längsrichtung aufgenäht. Die Verpressung erfolgte nach Konditionierung im Klimaschrank (20°C, 65 % rel. Luftfeuchte) axial und radial nach dem Tampax-Verfahren. Das Fasermaterial ließ sich problemlos verarbeiten und führte zu sehr guten Preßkörpern.

Beispiel 4

Nachweis der Unterdrückung bakteriellen Wachstums und der desodorierenden Wirkung:

Geprüfte Keime:  Staph. aureus
                 E. coli
                 Ps. aeruginosa
                 Proteus mirabilis
                 Candida albicans

. . .

0158092

- 12 -

Versuchsdurchführung:

Je 1 g Normalviskose (3.6 dtex, 30 mm) der Fa. Hoechst und mit Kupfer ausgerüstetes Fasermaterial gemäß Beispiel 1 und 2 wurden mit jeweils 10 ml CASO-Bouillon in Reagenzgläsern versetzt. Die so präparierten Reagenzgläser wurden mit jeweils 0,1 ml Keimsuspension (s.o.) - 24 h Bouillonkultur 1 : 100 verdünnt - geimpft und bei 37$^{O}$C inkubiert.

Ergebnis:

Im Falle der Normalviskose trat mit jedem Prüfstamm (s.o.) Wachstum ein, während bei den mit Kupfer ausgerüsteten Fasermaterialien auch nach 72 h in keinem Fall Wachstum festgestellt werden konnte. Gleichzeitig wurde auch nur im Falle der mit Normalviskose beschickten Ansätze eine Geruchsentwicklung beobachtet, die der normaler Bouillonkulturen der entsprechenden Keime entsprach. Bei den Ansätzen mit den Spezialwatten fehlte diese Geruchsbildung.

Beispiel 5

Prüfung der geruchsunterdrückenden Wirkung am Beispiel Menstrualblut

Menstrualblut wurde von verschiedenen Probandinnen mit Hilfe einer speziellen Vorrichtung gesammelt.

Jeweils 250 mg der Fasermaterialien nach Beispiel 1 und 2 sowie Normalviskose wurden mit 0,5 ml frischem Mentrualblut versetzt und bei 37$^{O}$C in einer feuchten Kammer inkubiert. Die Beurteilung der Geruchsentwicklung wurde unmittelbar nach Zugabe von Mentrualblut

. . .

- 13 -

sowie nach 2 h, 4h, 6 h und 24 h durchgeführt. Die Geruchsentwicklung wurde jeweils von 6 Prüferinnen bewertet. Zu allen Zeiten wurden die Proben der mit Kupfer ausgerüsteten Fasermaterialien wesentlich besser beurteilt. Die typische ekelerregende Geruchsbildung fehlte bei den Kupferwatten im Gegensatz zur Normalwatte; lediglich ein leichter kräuterartiger Geruch trat nach längeren Inkubationszeiten auf.

Beispiel 6

Prüfung der Geruchsentwicklung am Beispiel Rinderblut Jeweils 3 g Normalviskose und mit Kupfer ausgerüstetes Fasermaterial gemäß Beispiel 1 und 2 wurden mit 10 ml Rinderblut bei 37°C inkubiert. Die Ergebnisse entsprechen den unter 2. beschriebenen. Lediglich die Inkubationszeiten wurden vom Ausgangszustand des Blutes abhängig z.T. länger gewählt.

Beispiel 7

Prüfung als Saugmaterial in einer Slipeinlage

Aus dem Fasermaterial gemäß Beispiel 2 wurde nach üblichem Verfahren durch Auflösen an einer Versuchsanlage ein Watteband von 250 $g/m^2$ gefertigt, wobei eine Verfestigung des Kardenbandes durch Vernadelung erfolgte. Es wurden 50 x 150 mm (übliches Slipeinlagenformat) Abschnitte gestanzt, in ein Hüllvlies (20 $g/m^2$, Polypropylen) eingeschlagen und die Rückseite mit einer Polyethylenfolie abgedeckt. Entsprechend wurden Einlagen aus Normalviskose gefertigt. Die max. Aufnahmekapazität wurde in einem Bindenprüfgerät getestet.

- 14 -

Die Einlage wurde dabei um ein halbrundes Plastikteil gelegt, das unten mittig ein Loch besitzt, und mit einem Folienstreifen fixiert. Laufbewegungen wurden durch 2 Kunststoffbacken simuliert, die sich im Versuch mit 106 Takten/min hin und her bewegten. Der Druck auf die Einlage durch die beweglichen Backen betrug je Seite 160 g. Die Flüssigkeit wurde dann durch die Öffnung im Plastikteil bis zum seitlichen Durchdringen des 1. Tropfens aufgetropft (2 ml/min, Testflüssigkeit : Wasser). Zwischen den Mustern aus Normalviskose und mit Kupfer ausgerüstetem Fasermaterial konnten innerhalb der Fehlergrenzen keine Unterschiede festgestellt werden. Es wurden für beide Typen Werte von 18 - 22 ml/Einlage gemessen (Testzahl : jeweils 10 Stck.).

0158092

- 15 -

Patentansprüche

1. Sanitäre Hygienemittel, die ganz oder teilweise aus adsorbierendem, desodorierendem und mikrobistatischem Fasermaterial bestehen, dadurch gekennzeichnet, daß das Fasermaterial aus durch anionische, salzbildende Gruppen modifiziertenCellulosefasern besteht und über die anionischen Gruppen an die Faser gebundenes Kupfer enthält.

2. Sanitäre Hygienemittel nach Anspruch 1, dadurch gekennzeichnet, daß die Cellulosefaser über den Sauerstoff an die Anhydroglucoseeinheiten gebundene Gruppen der allgemeinen Formeln $-PO_3H^{(-)}$, $-(CH_2)_n-PO_3H^{(-)}$, $-(CH_2)_n-SO_3^{(-)}$ oder $-(CH_2)_n-COO^{(-)}$ tragen, in welchen n einen Wert von 1 - 3 hat und der Substitutionsgrad der Cellulose wenigstens so hoch ist, daß sie 0,1 bis 3,0 Gew.-% Kupfer, bezogen auf das Fasermaterial, binden kann.

3. Sanitäre Hygienemittel nach Anspruch 1 oder 2, dadurch gekennzeichnet, daß das Fasermaterial aus einer einheitlichen mit Carboxymethylgruppen modifizierten Viskosefaser besteht.

4. Sanitäre Hygienemittel nach Anspruch 3, dadurch gekennzeichnet, daß die modifizierte Viskosefaser bezüglich der Carboxymethylgruppen einen Substitutionsgrad von 0,01 bis 0,3 aufweist und der Gehalt an gebundenem Kupfer von 0,2 - 2,0 Gew.-%, bevorzugt von 0,6 - 1,6 Gew.-% des Fasermaterials ausmacht.

...

- 16 -

5. Sanitäre Hygienemittel nach Anspruch 1 - 4, dadurch
   gekennzeichnet, daß das Fasermaterial einen Faser-
   pH-Wert von 4 - 5 aufweist.

6. Sanitäre Hygienemittel nach Anspruch 1 - 5, dadurch
   gekennzeichnet, daß das Fasermaterial ein Wasserrückhaltevermögen von mehr als 80 % aufweist.

7. Verwendung von desodorierendem und mikrobistatischem
   Fasermaterial aus einer einheitlichen, mit Carboxymethylgruppen modifizierten Viskosefaser mit einem
   Substitutionsgrad von 0,01 bis 0,3 und mit einem Gehalt an gebundenem Kupfer von 0,2 - 2,0 Gew.-% für
   sanitäre Hygienemittel, insbesondere für Menstrualtampons, Binden und Slipeinlagen.

Europäisches Patentamt

**EUROPÄISCHER RECHERCHENBERICHT**

Ọ1 58092

Nummer der Anmeldung

EP 85 10 2235

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| X | FR-A-1 504 077 (THE CALICO PRINTERS' ASSOC.) * Zusammenfassung * | 1-7 | A 61 L 15/00 A 01 N 25/10 A 01 N 59/20 |
| X | FR-A-1 499 358 (MOSKOVSKY TEXTILNY INSTITUT) * Beispiel 2 * | 1 | |
| X | FR-A-1 499 788 (MOSKOVSKY TEXTILNY INSTITUT) * Beispiel 3 * | 1,2 | |
| A | US-A-2 856 330 (H.N. VAGENIUS) * Ansprüche 1-4 * | 1 | |
| D,A | GB-A-2 083 748 (LANDSTINGENS INKÖPSCENTRAL) * Ansprüche 1,6 * | 1 | RECHERCHIERTE SACHGEBIETE (Int. Cl.4) A 61 L A 01 N D 06 M |

Der vorliegende Recherchenbericht wurde für alle Patentansprüche erstellt.

| Recherchenort DEN HAAG | Abschlußdatum der Recherche 25-06-1985 | Prüfer PELTRE CHR. |
|---|---|---|